Europäisches Patentamt

European Patent Office

Office européen des brevets

(11) Veröffentlichungsnummer: **0 315 863 B1**

# EUROPÄISCHE PATENTSCHRIFT

(45) Veröffentlichungstag der Patentschrift: **18.11.93**

(51) Int. Cl.5: **C07C 211/52**

(21) Anmeldenummer: **88118103.6**

(22) Anmeldetag: **31.10.88**

(54) **Fluor enthaltende Trifluormethylaminobenzole und deren Herstellung.**

(30) Priorität: **09.11.87 DE 3737986**

(43) Veröffentlichungstag der Anmeldung:
**17.05.89 Patentblatt 89/20**

(45) Bekanntmachung des Hinweises auf die
Patenterteilung:
**18.11.93 Patentblatt 93/46**

(84) Benannte Vertragsstaaten:
**CH DE FR GB IT LI**

(56) Entgegenhaltungen:
**EP-A- 0 034 402**
**EP-A- 0 138 359**
**EP-A- 0 318 796**
**WO-A-83/00331**
**DE-A- 2 812 169**

(73) Patentinhaber: **BAYER AG**

**D-51368 Leverkusen(DE)**

(72) Erfinder: **Marhold, Albrecht, Dr.**
**Carl-Duisberg-Strasse 329**
**D-5090 Leverkusen 1(DE)**

**Beschreibung**

Die vorliegende Erfindung betrifft neue Verbindungen aus der Reihe Fluor enthaltender Trifluormethylaminobenzole und ein Verfahren zu deren Herstellung.

Bisher sind nur wenige Verbindungen aus der Reihe Fluor enthaltender Trifluormethylaminobenzole bekannt, beispielsweise 2,5-Difluor-3-amino-benzotrifluorid, 2-Chlor-3-amino-5-fluor-benzotrifluorid, 2- Fluor-3-amino-5-chlor-benzotrifluorid und 2,5,6-Trifluor-3-chlor-4-amino-benzotrifluorid (siehe EP-OS 0 138 359, US-PS 4 275 210, DE-OS 2 812 169 und Zh. Obshch, Khim. 33 (7), 2358-64 (1963)-).

Einige dieser bekannten Fluor enthaltender Trifluormethylaminobenzole sind nur auf sehr aufwendige Weise in einem vielstufigen Verfahren zugänglich, andere Verbindungen dieses Typs sind bisher überhaupt nicht beschrieben worden.

2-Amino-4-fluor-benzotrifluorid kann beispielsweise hergestellt werden, indem man 4-Fluorphthalsäureanhydrid mit Phosphorpentachlorid chloriert, anschließend mit Antimontrifluorid fluoriert, das erhaltene Trifluormethyl-fluor-benzoylfluorid in das entsprechende Amid überführt und aus diesem durch Hofmann-Abbau mit Hypobromit das entsprechende Anilin erhält (siehe Zh. Obshch. Khim. 33 (7), 2358-64 (1963)). Nachteilig ist die vielstufige Arbeitsweise.

Dieses Verfahren läßt sich nicht auf die Herstellung anderer Fluor enthaltender Trifluormethylaminobenzole übertragen, weil geänderte Substitutionsmuster geänderte Reaktivitäten bedeuten.

Es wurden nun neue Fluor enthaltende Trifluormethylaminobenzole gefunden, bei denen sich die Trifluormethylgruppe in 1-Stellung, die Aminogruppe in 4-Stellung

-   und ein Fluoratom in 2-Stellung und zusätzlich ein weiteres Fluoratom in 3- oder 6-Stellung oder zwei weitere Fluoratome in 5- und 6-Stellung
-   oder zwei Fluoratome in 3- und 5-Stellung
-   oder drei Fluoratome am aromatischen Ring

befinden.

Bei den vorstehenden Verbindungen kann es sich beispielsweise im einzelnen um 2,3-Difluor-4-amino-benzotrifluorid, 2,6-Difluor-4-amino-benzotrifluorid, 2,5,6-Trifluor-4-amino-benzotrifluorid, 3,5-Difluor-4-amino- benzotrifluorid, 2,5,6- Trifluor-4-amino-benzo-trifluorid oder 4-Amino-4,5,6-trifluor-benzotrifluorid und 2,4,6-Trifluor-3-amino-benzotrifluorid handeln.

Von diesen Verbindungen sind 2,3-Difluor-4-amino-benzotrifluorid, 2,6-Difluor-4-amino-benzotrifluorid, 3,5-Difluor-4-amino-benzotrifluorid und 2,5,6-Trifluor-4-amino-benzotrifluorid bevorzugt.

Die vorliegende Erfindung betrifft auch die Herstellung der erfindungsgemäßen Fluor enthaltenden Trifluormethylaminobenzole.

Ein bevorzugtes Verfahren zur Herstellung Fluor enthaltender Trifluormethylaminobenzole, bei denen sich die Trifluormethylgruppe in 1-Stellung, die Aminogruppe in 4-Stellung

-   und ein Fluoratom in 2-Stellung und zusätzlich ein weiteres Fluoratom in 3- oder 6-Stellung oder zwei weitere Fluoratome in 5- und 6-Stellung
-   oder zwei Fluoratome in 3- und 5-Stellung
-   oder drei Fluoratome am aromatischen Ring

befinden, ist dadurch gekennzeichnet, daß man entsprechende, Fluor enthaltende Trifluormethylbenzole nitriert und die so erhaltenen Fluor enthaltenden Trifluormethylnitrobenzole reduziert.

Die in dieses erfindungsgemäße Verfahren einzusetzenden Fluor und/oder Chlor enthaltenden, aber von Aminogruppen freien Trifluormethylbenzole sind bekannt (siehe z.B. J. Chem. Soc. C, (8) 1547-1549 (1971) und Can. J. Chem. 57 (7), 807-812 (1979)).

Die Nitrierung kann mit üblichen Nitriermitteln durchgeführt werden, beispielsweise mit Gemischen aus Salpeter- und Schwefelsäure. Die Temperatur kann dabei beispielsweise im Bereich von 0 bis 80°C liegen, vorzugsweise liegt sie bei 20 bis 50°C. Das Nitriermittel kann man beispielsweise in Mengen einsetzen, bei denen im Reaktionsgemisch 0,8 bis 1,5 Mole Nitrieragenz pro Mol Ausgangsverbindung gebildet werden. Vorzugsweise setzt man soviel Nitriermittel ein, daß sich 1 bis 1,1 Mole Nitrieragenz pro Mol Ausgangsverbindung bilden. Die Nitrierung kann gegebenenfalls in Gegenwart eines inerten organischen Lösungsmittels durchgeführt werden. Geeignet ist beispielsweise Methylenchlorid.

Die daran anschließende Reduktion kann chemisch, d.h. beispielsweise mit reduzierend wirkenden Metallen oder Metallsalzen durchgeführt werden. Geeignet sind beispielsweise Eisen, Zink, Zinn, Zinn(II)-chlorid und Titan(III)-chlorid. Derartige Reduktionsmittel werden vorzugsweise in der stöchiometrisch erforderlichen Menge eingesetzt. Für eine derartige Reduktion können die Nitroverbindungen z.B. so eingesetzt werden, wie sie bei der Nitrierung anfallen oder in gereinigter Form.

Die Reduktion kann man auch katalytisch mit Wasserstoff durchführen, wobei z.B. Katalysatoren eingesetzt werden können, die Metalle enthalten oder daraus bestehen. Geeignet sind beispielsweise die Metalle VIII. Nebengruppe des Periodensystems der Elemente, insbesondere Palladium, Platin und Nickel. Die Metalle können in elementarer Form oder in Form von Verbindungen vorliegen, sowie in besonders aktivierten Formen, z.B. in Form von Raney-Metallen oder als Metall oder Metallverbindung aufgebracht auf Trägermaterialien. Bevorzugt ist Raney-Nickel, Palladium auf Kohle und Palladium auf Aluminiumoxid.

Vorzugsweise wird die katalytische Reduktion in Gegenwart eines Lösungsmittels durchgeführt. Geeignet sind beispielsweise Alkohole und Ether, wie Methanol, Ethanol und Tetrahydrofuran. Die katalytische Reduktion kann beispielsweise bei Temperaturen im Bereich 10 bis 60°C und beispielsweise bei Wasserstoffdrucken im Bereich 1 bis 100 bar durchgeführt werden. Überschüsse an Wasserstoff sind im allgemeinen nicht kritisch.

Für die katalytische Reduktion werden vorzugsweise säurefreie Nitroverbindungen eingesetzt. Nach der Herstellung sind sie also gegebenenfalls von Säuren zu befreien, z.B. durch Waschen mit Wasser oder Neutralisation mit einer Base.

Die Aufarbeitung des nach der chemischen Reduktion oder der katalytischen Hydrierung vorliegenden Reaktionsgemisches kann beispielsweise so erfolgen, daß man zunächst gegebenenfalls vorhandene feste Bestandteile abfiltriert und das Filtrat, gegebenenfalls nach einer Wäsche mit Wasser, destilliert. Falls als Reaktionsprodukt ein Gemisch aus Isomeren anfällt kann man diese gegebenenfalls durch Feindestillation trennen.

In einer Variante der zuvor beschriebenen katalytischen Reduktion wird in Gegenwart einer Base gearbeitet, beispielsweise in Gegenwart von Hydroxiden oder Carbonaten der Alkalimetalle oder tertiären Aminen. Bevorzugt sind tertiäre Amine wie Triethylamin oder Pyridin. Mit jeweils einem Äquivalent Base pro Mol der in die katalytische Reduktion eingesetzten Nitroverbindung kann aus letzterer während der katalytischen Reduktion zusätzlich ein Äquivalent Chloratome abgespalten werden. Man kann so beispielsweise aus einem Fluor und Chlor enthaltenden Nitrobenzotrifluorid ein von Chlor freies, Fluor enthaltendes Aminobenzotrifluorid erhalten.

Ein weiteres Verfahren speziell zur Herstellung von Fluor enthaltenden Trifluormethylaminobenzolen, bei denen sich die Trifluormethylgruppe in 1-Stellung, die Aminogruppe in 4-Stellung und ein Fluoratom in 2-Stellung und zusätzlich ein weiteres Fluoratom in 3- oder 6-Stellung oder zwei weitere Fluoratome in 5- und 6-Stellung
- oder zwei Fluoratome in 3- und 5-Stellung
- oder drei Fluoratome am aromatischen Ring

befinden, ist dadurch gekennzeichnet, daß man entsprechende Fluor enthaltende, 4-Halogen-trifluormethylbenzole bei erhöhtem Druck mit Ammoniak in Gegenwart eines organischen Lösungsmittels umsetzt.

Die in dieses Verfahren einzusetzenden 4-Halogen-trifluormethylbenzole sind bekannt (siehe Izv. Sib. Otd. Akad. Nauk. SSSR, Ser. Khim. Nauk., (2), 133- 141 (1977)), Vorzugsweise handelt es sich dabei um 2- und/oder 4-Chlor-trifluormethylbenzole oder um 2-und/oder 4-Fluor-trifluormethylbenzole.

Das Ammoniak kann in flüssiger oder gasförmiger Form zugesetzt werden, z.B. in Substanz (gasförmig oder flüssig) oder als wäßrige Lösung. Pro Mol in 2- und/oder 4-Stellung durch $NH_2$-Gruppen zu ersetzende Halogenatome kann man beispielsweise 1 bis 10 Mol Ammoniak verwenden. Bevorzugt beträgt diese Menge 3 bis 8 Mol. Geeignete Temperaturen für diese Umsetzung sind beispielsweise solche im Bereich von 80 bis 160°C, bevorzugt sind solche im Bereich 100 bis 130°C. Die Umsetzung kann unter dem sich im geschlossenen Gefäß bei Reaktionstemperatur einstellenden Eigendruck des Ammoniaks durchgeführt werden, der beispielsweise im Bereich von 10 bis 20 bar liegen kann. Man kann auch höhere Drucke anwenden, beispielsweise solche bis zu 100 bar.

Als Lösungsmittel für diese Umsetzung sind inerte oder weitgehend inerte organische Lösungsmittel der verschiedensten Art einsetzbar. Beispielsweise kommen in Frage: Alkohole, Ether, Sulfone und aromatische Kohlenwasserstoffe.

Aus dem nach der Umsetzung vorliegenden Reaktionsgemisch kann man das oder die gewünschten Reaktionsprodukte z.B. erhalten, indem man zunächst abkühlt und den Druck entspannt, dann das Lösungsmittel entfernt und anschließend eine Destillation, vorzugsweise unter vermindertem Druck, durchführt.

Die erfindungsgemäßen Fluor enthaltenden Trifluormethylaminobenzole sind wertvolle Zwischenprodukte. Beispielsweise kann man sie mit Acrylnitril unter Diazotierungsbedingungen umsetzen, so $\alpha$-Chlor-$\beta$-phenyl-propionitrile erhalten, deren Phenylteil (mit Ausnahme der $NH_2$-Gruppe) in gleicher Weise substituiert ist wie im jeweils eingesetzten erfindungsgemäßen Produkt, diese durch Dehydrohalogenierung in das entsprechende Zimtsäurenitril überführen und daraus durch Umsetzung mit Sulfonylmethylisocyaniden 3-Cyano-4-phenyl-pyrrole gewinnen, deren Phenylteil (mit Ausnahme der $NH_2$-Gruppe) in gleicher Weise substituiert ist, wie im jeweils eingesetzten erfindungsgemäßen Produkt, Derartige 3-Cyano-4-phenylpyrrole weisen eine besonders gute Wirksamkeit als Fungizide auf, insbesondere zum Schutz von Pflanzen gegen Plasmodiophoromycetes, Oomycetes, Chrytridiomycetes, Zygomycetes, Ascomycetes, Basidiomycetes und Deuteromycetes.

Beispiele

Beispiel 1

In einem Edelstahlautoklaven wurden 200 ml Tetrahydrofuran und 50 g 2,4,6-Trifluorbenzotrifluorid vorgelegt und 30 ml flüssiger Ammoniak aufgedrückt. Anschließend wurde unter Rühren für 6 Stunden auf 120°C erhitzt. Nach Abkühlen und Entspannen wurde die Reaktionsmischung einer fraktionierten Destillation unterworfen. Bei einem Siedepunkt von 57 bis 58°C bei 12 mbar wurden 15 g 2-Amino-4,6-difluor-benzotrifluorid erhalten. Nach einem kleinen Zwischenlauf gingen bei einem Siedepunkt von 103 bis 105°C bei 16 mbar 32 g 2,6-Difluor-4-amino-benzotrifluorid über. Der Schmelzpunkt des erhaltenen 2,6-Difluor-4-amino-benzotrifluorids lag bei 66°C.

### Beispiel 2

In einem Edelstahlautoklaven wurden 500 g 3,4,5-Trifluor-benzotrifluorid vorgelegt, 2000 ml Tetrahydrofuran zugegeben und 150 g flüssiger Ammoniak aufgedrückt. Unter Rühren wurde der Autoklav für 5 Stunden auf 120 bis 130°C erhitzt, dann abgekühlt und bei 20°C entspannt. Durch Destillation wurden neben dem Lösungsmittel und Ausgangsmaterial 272 g 3,5-Difluor-4-aminobenzotrifluorid mit einem Siedepunkt von 58 bis 60°C bei 16 mbar erhalten.

### Beispiel 3

In einem Autoklaven wurden 200 g 2,3,4-Trifluorbenzotrifluorid in 500 ml Tetrahydrofuran vorgelegt und 60 ml flüssiger Ammoniak aufgedrückt. Nach 6 Stunden Rühren bei 130°C (maximaler Druck 18 bar) wurde abgekühlt und entspannt. Durch Destillation wurden bei einem Siedepunkt von 60 bis 64°C bei 26 mbar 72 g 2-Amino-3,4-difluor-benzotrifluorid und bei einem Siedepunkt von 92 bis 93°C bei 26 mbar 92 g 2,3-Difluor-4-amino-benzotrifluorid erhalten.

### Beispiel 4

In einem Edelstahlautoklaven mit 0,7 l Inhalt wurden 110 g 2,3,4,6-Tetrafluor-benzotrifluorid in 200 ml Tetrahydrofuran vorgelegt und 30 ml flüssiger Ammoniak aufgedrückt. Anschließend wurde für 3 Stunden auf 100°C erhitzt, wobei sich ein maximaler Druck von 10 bar einstellte. Nach dem Abkühlen wurde entspannt und die flüssige Phase einer fraktionierten Destillation unterworfen. Zuerst wurden bei einem Siedepunkt von 47 bis 48°C bei 8 mbar 42 g 2-Amino-3,4,6-Trifluor-benzotrifluorid erhalten, dann nach einem Zwischenlauf bei einem Siedepunkt von 75 bis 78°C bei 8 mbar 46 g 2,5,6-Trifluor-4-amino-benzotrifluorid.

### Beispiel 5

In einem Autoklaven wurden 225 g 2,4,5-Trifluorbenzotrifluorid in 600 ml Tetrahydrofuran vorgelegt und 100 ml flüssiger Ammoniak aufgedrückt. Unter Rühren mit 400 U/min. wurde für 8 Stunden auf 140°C geheizt und dann auf 20°C abgekühlt. Nach Entspannen des nicht verbrauchten Ammoniaks wurde der Ansatz destilliert. Im Vorlauf wurden 37 g 4,5-Difluor-2-aminobenzotrifluorid erhalten. Die Hauptmenge destilliert bei 78°C/18 mbar und ist nach NMR-spektroskopischen Befunden das 2,5-Difluor-4-aminobenzotrifluorid ($n_D^{20}$ : 1.4560) mit einer Ausbeute von 154 g.

### Patentansprüche

1. Fluor enthaltende Trifluormethylaminobenzole, dadurch gekennzeichnet, daß sich die Trifluormethylgruppen in 1-Stellung, die Aminogruppe in 4-Stellung
   - und ein Fluoratom in 2-Stellung und zusätzlich ein weiteres Fluoratom in 3- oder 6-Stellung oder zwei weitere Fluoratome in 5-und 6-Stellung
   - oder zwei Fluoratome in 3- und 5-Stellung
   - oder drei Fluoratome am aromatischen Ring

   befinden.

2. Fluor enthaltende Trifluormethylaminobenzole nach Anspruch 1, dadurch gekennzeichnet, daß es sich um 2,3-Difluor-4-amino-benzotrifluorid, 2,6-Difluor-4-amino-benzotrifluorid, 2,5,6-Trifluor-4-amino-benzotrifluorid, 3,5-Difluor-4-amino-benzotrifluorid, 2,5,6-Trifluor-4-amino- benzotrifluorid oder 4-Amino-4,5,6-trifluor-benzotrifluorid handelt.

3. Verfahren zur Herstellung von Fluor enthaltenden Trifluormethylaminobenzolen, bei denen sich die Trifluormethylgruppe in 1-Stellung, die Aminogruppe in 4-Stellung und
   - und ein Fluoratom in 2-Stellung und zusätzlich ein weiteres Fluoratom in 3- oder 6-Stellung oder zwei weitere Fluoratome in 5-und 6-Stellung
   - oder zwei Fluoratome in 3- und 5-Stellung
   - oder drei Fluoratome am aromatischen Ring

   befinden, dadurch gekennzeichnet, daß man entsprechende Fluor enthaltende Trifluormethylbenzole nitriert und die so erhaltenen Fluor enthaltenden Trifluormethylnitrobenzole reduziert.

4. Verfahren nach Anspruch 3, dadurch gekennzeichnet, daß man die Nitrierung mit einem Gemisch aus Salpeter- und Schwefelsäure bei Temperaturen im Bereich von 0 bis 80°C durchführt.

5. Verfahren nach Ansprüchen 3 und 4, dadurch gekennzeichnet, daß man die Reduktion chemisch oder katalytisch mit Wasserstoff durchführt.

6. Verfahren nach Ansprüchen 3 bis 5, dadurch gekennzeichnet, daß man die Reduktion mit reduzierend wirkenden Metallen oder Metallsalzen durchführt.

7. Verfahren nach Ansprüchen 3 bis 5, dadurch gekennzeichnet, daß man die Reduktion katalytisch mit Wasserstoff durchführt, dabei Katalysatoren verwendet die Metalle der VIII. Nebengruppe des periodischen Systems der Elemente in metallischer Form oder in Form von Verbindungen enthalten und bei Temperaturen im Bereich von 10 bis 60°C und bei Wasserstoffdrucken im Bereich 1 bis 100 bar durchführt.

8. Verfahren nach Ansprüchen 3 bis 5 und 7, dadurch gekennzeichnet, daß man die Reduktion in Gegenwart einer Base durchführt.

9. Verfahren zur Herstellung von Fluor enthaltenden Trifluormethylaminobenzolen, bei denen sich die Trifluormethylgruppe in 1-Stellung, die Aminogruppe in 4-Stellung
- und ein Fluoratom in 2-Stellung und zusätzlich ein weiteres Fluoratom in 3- oder 6-Stellung oder zwei weitere Fluoratome in 5-und 6-Stellung
- oder zwei Fluoratome in 3- und 5-Stellung
- oder drei Fluoratome am aromatischen Ring

befinden, dadurch gekennzeichnet, daß man entsprechende Fluor enthaltende, 4-Halogentrifluormethylbenzole bei erhöhtem Druck mit Ammoniak in Gegenwart eines organischen Lösungsmittels umsetzt.

## Claims

1. Trifluoromethylaminobenzenes containing fluorine, characterised in that the trifluoromethyl groups are situated in the 1-position and the amino group is situated in the 4-position
- and a fluorine atom is situated in the 2-position and additionally a further fluorine atom is situated in the 3- or 6-position or two further fluorine atoms are situated in the 5- and 6-position
- or two fluorine atoms are situated in the 3- and 5-position
- or three fluorine atoms are situated on the aromatic ring.

2. Trifluoromethylaminobenzenes containing fluorine according to Claim 1, characterised in that they are 2,3-difluoro-4-amino-benzotrifluoride, 2,6-difluoro-4-amino-benzotrifluoride, 2,5,6-trifluoro-4-amino-benzotrifluoride, 3,5-difluoro-4-amino-benzotrifluoride, 2,5,6-trifluoro-4-amino-benzotrifluoride or 4-amino-4,5,6-trifluoro-benzotrifluoride.

3. Process for the preparation of trifluoromethylaminobenzenes containing fluorine, in which the trifluoromethyl group is situated in the 1-position and the amino group is situated in the 4-position
- and a fluorine atom is situated in the 2-position and additionally a further fluorine atom is situated in the 3- or 6-position or two further fluorine atoms are situated in the 5- and 6-position
- or two fluorine atoms are situated in the 3- and 5-position
- or three fluorine atoms are situated on the aromatic ring,

characterised in that corresponding trifluoromethylbenzenes containing fluorine are nitrated and the fluorine-containing trifluoromethylnitrobenzenes thus obtained are reduced.

4. Process according to Claim 3, characterised in that the nitration is carried out with a mixture of nitric and sulphuric acid at temperatures in the range from 0 to 80°C.

5. Process according to Claims 3 and 4, characterised in that the reduction is carried out chemically or catalytically using hydrogen.

6. Process according to Claims 3 to 5, characterised in that the reduction is carried out using reducing metals or metal salts.

7. Process according to Claims 3 to 5, characterised in that the reduction is carried out catalytically using hydrogen, using catalysts which contain metals of subgroup VIII of the Periodic Table of the Elements in metallic form or in the form of compounds and at temperatures in the range from 10 to 60°C and at hydrogen pressures in the range 1 to 100 bar.

8. Process according to Claims 3 to 5 and 7, characterised in that the reduction is carried out in the presence of a base.

**9.** Process for the preparation of trifluoromethylaminobenzenes containing fluorine, in which the trifluoromethyl group is situated in the 1-possition and the amino group is situated in the 4-position

- and a fluorine atom is situated in the 2-position and additionally a further fluorine atom is situated in the 3- or 6-position or two further fluorine atoms are situated in the 5- and 6-position
- or two fluorine atoms are situated in the 3- and 5-position
- or three fluorine atoms are situated on the aromatic ring,

characterised in that corresponding fluorine-containing, 4-halogeno-trifluoromethyl-benzenes are reacted with ammonia at elevated pressure in the presence of an organic solvent.

**Revendications**

**1.** Trifluorométhylaminobenzènes contenant du fluor, caractérisés en ce que les groupes trifluorométhyle se trouvent en position 1, les groupes amino en position 4

- et un atome de fluor est en position 2 et en outre, un autre atome de fluor est en position 3 ou 6 ou deux autres atomes de fluor sont en positions 5 et 6
- ou bien deux atomes de fluor sont en positions 3 et 5

ou bien trois atomes de fluor sont sur le noyau aromatique.

**2.** Trifluorométhylaminobenzènes contenant du fluor suivant la revendication 1, caractérisés en ce qu'il s'agit du fluorure de 2,3-difluoro-4-amino-benzényle, du fluorure de 2,6-difluoro-4-amino-benzényle, du fluorure de 2,5,6-trifluoro-4-amino-benzényle, du fluorure de 3,5-difluoro-4-amino-benzényle, dufluorure de 2,5,6-trifluoro-4-amino-benzényle ou du fluorure de 4-amino-4,5,6-trifluorobenzényle.

**3.** Procédé de production de trifluorométhylaminobenzènes contenant du fluor, dans lesquels le groupe trifluorométhyle se trouve en position 1, le groupe amino est en position 4 et

- un atome de fluor est en position 2 et en outre, un autre atome de fluor est en position 3 ou 6 ou bien deux autres atomes de fluor sont en positions 5 et 6
- ou bien deux atomes de fluor se trouvent en positions 3 et 5,
- ou bien trois atomes de fluor sont sur le noyau aromatique,

caractérisé en ce qu'on effectue la nitration de trifluorométhylbenzènes correspondants contenant du fluor et on réduit les trifluorométhylnitrobenzènes contenant du fluor ainsi obtenus.

**4.** Procédé suivant la revendication 3, caractérisé en ce qu'on conduit la nitration avec un mélange d'acide nitrique et d'acide sulfurique à des températures comprises dans la plage de 0 à 80°C.

**5.** Procédé suivant les revendications 3 et 4, caractérisé en ce qu'on conduit la réduction chimiquement ou catalytiquement avec de l'hydrogène.

**6.** Procédé suivant les revendications 3 à 5, caractérisé en ce qu'on conduit la réduction avec des métaux ou des sels métalliques à action réductrice.

**7.** Procédé suivant les revendications 3 à 5, caractérisé en ce qu'on conduit la réduction catalytiquement avec de l'hydrogène, on utilise alors des catalyseurs qui contiennent des métaux du sous-groupe VIII du Système Périodique des Eléments sous la forme métallique ou sous forme de composés et on opère à des températures comprises dans la plage de 10 à 60°C et à des pressions d'hydrogène comprises dans la plage de 1 à 100 bars.

**8.** Procédé suivant les revendications 3 à 5 et 7, caractérisé en ce qu'on conduit la réduction en présence d'une base.

**9.** Procédé de production de trifluorométhylaminobenzènes contenant du fluor, dans lesquels le groupe trifluorométhyle se trouve en position 1, le groupe amino se trouve en position 4

- et un atome de fluor se trouve en position 2 et en outre, un autre atome de fluor se trouve en position 3 ou 6 ou bien deux autres atomes de fluor se trouvent en positions 5 et 6
- ou bien deux atomes de fluor se trouvent en positions 3 et 5
- ou bien trois atomes de fluor sont sur le noyau aromatique,

caractérisé en ce qu'on fait réagir des 4-halogéno-triflurométhylbenzènes correspondants contenant du fluor à une pression élevée avec de l'ammoniac en présence d'un solvant organique.